# EUROPEAN PATENT SPECIFICATION

(11) **EP 2 699 619 B1**
(45) Date of publication and mention of the grant of the patent: **13.12.2017**
(21) Application number: 12721432.8
(22) Date of filing: 20.04.2012
(51) Int. Cl.: C08G 69/48, C08J 3/24, A61K 49/18, C12N 11/02, A61L 15/32

(54) **CROSS-LINKED POLY-E-LYSINE PARTICLES**
VERNETZTE POLY-E-LYSINTEILCHEN
PARTICULES D'EPSILON-POLYLYSINE RÉTICULÉE

(30) Priority: 20.04.2011 GB 201106742
(43) Date of publication of application: 26.02.2014
(73) Proprietor: Spheritech Ltd, Runcorn, Cheshire WA7 4QX (GB)
(72) Inventor: WELLINGS, Donald, Runcorn Cheshire WA7 4QX (GB)
(74) Representative: Geary, Stephen
(86) International application number: PCT/EP2012/057264
(87) International publication number: WO 2012/143508

(56) References cited:
- EP-A1- 2 210 917
- EP-A2- 1 967 217
- WO-A1-90/13256
- WO-A2-2007/028607
- JP-A- 11 152 330
- US-A1- 2003 103 931
- US-A1- 2010 161 021
- DATABASE WPI Week 200701 Thomson Scientific, London, GB; AN 2007-004399 XP002680959, -& JP 2006 307004 A (CHISSO CORP) 9 November 2006 (2006-11-09)
- DATABASE WPI Week 200382 Thomson Scientific, London, GB; AN 2003-882240 XP002680960, -& JP 2003 171464 A (CHISSO CORP) 20 June 2003 (2003-06-20)
- DATABASE WPI Week 200382 Thomson Scientific, London, GB; AN 2003-882294 XP002680961, -& JP 2003 176353 A (CHISSO CORP) 24 June 2003 (2003-06-24)

## Description

This invention relates to a polymeric support, a method of preparing the support and the use of the support in a solid phase process. In particular, the invention relates to a polymeric support comprising cross-linked poly-ε-lysine. The support is useful in a wide range of physical and chemical processes especially where interaction with a substrate is required for example solid phase synthesis, solid phase extraction, solid phase reagents, immobilization of species , cell culture, catalysis, chromatography and in medical diagnostics.

Poly-ε-lysine is a naturally occurring short chain polydispersed polyamide consisting of the amino acid lysine linked through amide bonds between the carboxyl and the epsilon (ε) amino group. This structure is unusual in that the amide bonds are formed between the carboxyl and ε amino groups of lysine whereas a normal peptide bond between amino acids in a peptide and traditionally employed poly-lysine, is formed between the carboxyl and α amino groups. The polydispersity in naturally occurring poly-ε-lysine is typically between 25-35 amino acids. In traditional commercially available poly-lysine the peptide has a much broader poorly controlled polydispersity typically containing anything from 5-500 amino acids.

Cross-linked poly-ε-lysine structurally has a more elastic nature than traditional poly-lysine due to the greater chain length between the amide bonds. Structurally poly-ε-lysine is effectively an α-amino Nylon 5.

Poly-ε-lysine is currently manufactured by bacterial fermentation on a large scale as a food preservative. It is inexpensive and readily available in commercial quantities. In this invention we describe the cross-linking of poly-ε-lysine to form insoluble polymeric supports that have applications across a range of technologies. These include solid phase peptide synthesis, solid phase oligonucleotide synthesis, solid phase extraction, solid phase reagents, immobilization of species, cell culture, catalysis, chromatography, slow release of agrochemicals, and slow release of pharmaceuticals, wound care, regenerative medicine and medical diagnostics.

Solid support materials useful in solid phase synthetic processes are known. A wide range of physical and chemical processes employ solid support materials including by way of example synthesis of organic molecules, in particular peptides and oligonucleotides, immobilization of species, support of catalysts, ion exchange, extraction of species from a material, diagnostics and chromatography.

Typically, multi-stage synthesis of an organic molecule involves numerous isolation steps to separate intermediates, produced at each stage, before progressing to the subsequent stage. These processes are often time-consuming, expensive and may be inefficient as regards yield. The intermediates often require purification to remove excess reagents and reaction by-products and procedures such as precipitation, filtration, bi-phase solvent extraction; solid phase extraction, crystallization and chromatography may be employed.

Solid phase synthesis offers some advantages over solution phase synthesis. For example, isolation procedures used in solution phase synthesis may to some extent be avoided by reversibly attaching the target molecule to a solid support. Excess reagents and some of the side-products may be removed by filtration and washing of the solid support. The target molecule may be recovered in essentially quantitative yield in some processes which is typically particularly difficult in solution phase synthesis. In addition, the time required to perform operations on a solid support is typically much less than that required carrying out the equivalent stage in a solution phase synthesis.

Immobilization of species in a range of processes is also known. For example, polymer supports are commonly used for the immobilization of catalysts for use in traditional organic chemistry including chemo and bio catalysis. Immobilized enzymes may be employed to perform organic chemical reactions or for chiral resolution, for example the use of immobilized Penicillin amidase for the resolution of secondary alcohols (E. Baldaro et al. Tet. Asym. 4, 1031, (1993) and immobilized Penicillin G amidase is also used for the hydrolysis of Benzylpenicillin in the manufacture of Amoxicillin (Carleysmith, S. W. and Lilly, M.D. Biotechnol. Bioeng., 21, 1057-73, 1979).

Solid supports are also used to immobilize biological macromolecules for medical and diagnostic applications. This includes immobilization of proteins, monoclonal and polyclonal antibodies. Cell culture is commonly carried out on solid supports with specific surface characteristics and morphology. Immobilized enzymes on the supports can also be employed as sensors to generate a signal. An example is the detection of glucose by the glucose oxidase/peroxidase coupled enzyme system, in which the presence of glucose generates hydrogen peroxide which in turn is the substrate for peroxidase for the oxidation of a wide variety of substrates to provide a coloured, fluorescent or luminescent signal.

A variety of fluors whose fluorescence is sensitive to specific cations or anions may be utilized to indicate concentrations of specific ions including hydrogen ions for pH measurement.

Polymeric particles are often used in chromatography where the solid supports are termed stationary phases. In certain modes of chromatography the cost of stationary phases may be restrictive. In other modes the physical nature of the stationary phase can reduce the effectiveness of the technology. For instance, the soft polymers often used for affinity, ionexchange and gel permeation chromatography cannot be used at high flow rates because of the deformable nature of the particles. The rigid macroporous polymers used for many other modes of chromatography can often be mechanically friable and subsequently suffer from a short lifetime.

The application of solid supports or stationary phases in chromatographic separations is very extensive for example complex high-technology separations used in the pharmaceutical and biotechnology industry and larger scale processes used in the mining industry. Some of the pharmaceutical industry's most valuable drugs are purified by preparative chromatography and improved chromatographic separation would be technically beneficial and economically advantageous. In the mining and precious metal recovery industry a large portion of the world's palladium, a critical component in a wide range of industrial applications and processes including catalytic converters and manufacture of high value products, may be refined using immobilized crown ethers (Traczyk, F.P.; Bruening, R.L.; Izatt, N.E. "The Application of Molecular Recognition Technology (MRT) for Removal and Recovery of Metal Ions from Aqueous Solutions"; In Fortschritte in der Hydrometallurgie; 1998, Vorträge beim 34. Metallurgischen Seminar des Fachausschusses fuer Metallurgische Aus-und Weiterbildung der GDMB; 18-20 November 1998; Goslar).

The use of polymeric particles in solid phase extraction and in the preparation of solid phase reagents is also known in the chemical, pharmaceutical and biotechnology industry.

Known solid phase supports generally comprise polymer particles of a particular size and physical nature to suit the application. For ease of use these polymer particles are often spherical and have a defined particle size distribution. The spherical nature of the particles improves the flow and filtration characteristics of the polymer. Although the uses of solid supports have operational advantages there are disadvantages to the solid phase approach.

In peptide synthesis, polystyrene is widely employed as a polymer support for supporting the growing peptide and is relatively low-cost, widely available and provides acceptable performance in peptide synthesis. Other commercially available supports commonly used for solid phase synthesis of peptides and oligonucleotides may be expensive, for example due to the complex manufacturing processes. Microporous polymers and macroporous polymers are generally used. Microporous polymers have a relatively low level of cross-linker which allows the polymer particles to solvate and consequently swell in suitable solvents. Macroporous polymers often have a high level of cross-linker in the polymer matrix and contain large pores. These polymer particles are generally rigid and have good flow characteristics and are suitable for use in packed columns.

A range of polymers are known including those in WO2007/028607 and EP1967217A2 which relate to nanoparticles and use of poly-ε-lysine as a coating on the particles to impart antimicrobial activity. JP2006/307004 refers to hydrogels. US2010/161021 refers to cross-linking of a polysaccharide for applications in myocardial patch. Polylysine is referred to in generic terms but no reference is made to poly-ε-lysine. US2003/103931 refers to poly-ε-lysine cross-linked with epichlorohydrin or bis-epoxy compounds. JP11152330 describes cross-linked. JP2003171464 and JP2003176353 relate to manufacture of a soluble polymer. EP2210917 relates to poly-L-lysine cross-linked by aldehyde. WO9013256 relates to poly-α-lysines containing 50-100 lysine groups.

A need remains to find an alternative or cost-effective polymer suitable for use as a support in a wide range of applications. We have now found that a cross-linked polo-ε-lysine polymer provides an excellent combination of characteristics, may be tailored according to the desired properties by appropriate selection of the cross-linker and may be employed in a wide-range of applications cost-effectively.

In a first aspect, the invention provides a cross-linked poly-ε-lysine polymer comprising poly-ε-lysine and a cross linker linked by amide bonds wherein the cross linker comprises two or more carboxylic acid groups and an aliphatic chain linking the two or more groups adapted to react with an alpha carbon amine of poly-ε-lysine.

The cross-linked poly-ε-lysine polymer is particularly useful in providing a support for a wide-range of applications especially where the cross-linking provides a polymer which is insoluble in water and other solvents. Where lower levels of cross-linking are employed, the polymer may be soluble in water and this provides advantage in certain applications as described below. Suitably the polymer comprises poly-ε-lysine and a cross linker linked by amide bonds.

The polymer may be in any suitable form including a non-particulate macro form for example a sheet, article, fibre, or in particulate form.

The poly-ε-lysine component of the cross-linked poly-ε-lysine is the principle component of the cross-linked polymer. The cross-linker acts to bond poly-ε-lysine polymers together such that the poly-ε-lysine polymers provide a structure, for example a lattice for use in a range of applications including support for synthesis of organic molecules, for example polypeptides and polynucleotides, chromatography, use as a support for functional materials as described herein below.

The cross-linker may have three or more functional groups to link to the same number of poly-ε-lysine polymer chains or fewer chains but with multiple links to one or more such chains. The cross-linker may contain other functional groups which do not participate in the cross-linking reaction and remain available for reaction with other species during use of the cross-linked polymer.

The level of cross-linking in the polymeric support can be used to control the physical form and chemical reactivity of the final cross-linked poly-ε-lysine. Introduction of high levels of cross-linking will produce rigid structures suitable for the preparation of macroporous polymer supports, whereas a low level of cross-linking will produce softer more microporous materials. The amine functionality is high with low levels of cross-linking, which can be readily tailored by controlled capping. The handling, solvation and physical properties can also be defined by the type of cross-linker introduced.

Suitably the cross-linker comprises a specific compound or group of compounds. The cross-linker may comprise a repeat unit and be polydisperse however, the polydispersity is narrow to ensure appropriate control over the lattice structure formed upon cross-linking. The crosslinker comprises at least two functional groups capable of reacting with an alpha carbon amine of poly-ε-lysine. Examples of common functional groups suitable for this purpose include carboxylic acids.

Amide bonds are biodegradable and the cross-linked polymer of the invention and supports comprising it are especially useful in applications in which biodegradability is important. Further, amide bonds may be metabolized for example by enzymes including proteases, as well as being biodegradable and are particularly advantageous for use in medical applications and especially medical use on or in the human or animal body. The polymers and supports of the invention provide benefit in medical applications where the polymer or support suitably degrades over time thereby avoiding the need for procedures to remove the support after its function has been completed.

In an especially preferred embodiment, the bonds between the cross-linker and the polo-ε-lysine comprise amide bonds, preferably at least 1% of the epsilon amine groups are cross-linked, more preferably at least 50%, desirably at least 90% and especially substantially all of the bonds are amide bonds.

In a further preferred embodiment, the polymer is lightly cross-linked and from 1 to up to 50%, 1 to 20% and 1 to 10% of the epsilon amine groups are cross-linked. Lightly cross-linked polymers and supports of the invention are especially useful in synthesis of organic species, for example peptides and nucleotide sequences, and in delivery of active species, for example pharmaceutically active agents.

Accordingly, in a preferred embodiment, the cross-linker may comprise a polyacid. In a preferred embodiment, the cross-linker comprises a compound of formula X[CO₂H]ₙ where n is 2 or more, preferably 2 to 6 more preferably 2 to 4 and X is a hydrophobic or hydrophilic linking group, preferably aliphatic. Suitably group X has a molecular weight of 14 to 250, more preferably 28 to 140 excluding any functional substituents on the linking group.

X may contain heteroatoms for example oxygen and nitrogen as part of the backbone of the linking group and may contain functional groups for reaction with other species during use of the cross-linked poly-ε-lysine polymer.

The aliphatic chain linking the acid groups may be hydrophilic, preferably a bis-carboxy-polyalkylene glycol, for example bis-carboxy-polyethyleneglycol, or the aliphatic chain may be hydrophobic providing a hydrophobic cross-linker, for example, sebacic acid provides a more lipophilic support. The cross-linker may be derived from a precursor material, for example an anhydride. Examples of other suitable cross-linkers include nitrilotriacetic acid, glutaric acid and HOOCCH₂CH₂CONHCH₂CH₂OCH₂CH₂OCH₂CH₂OCH₂CH₂NHCO CH₂CH₂COOH. Suitably, the cross linker is selected according to its solvation characteristics such that upon reaction with the poly-ε-lysine polymer, the polymer and cross-linker are contained within a disperse phase comprising a solvent for the cross-linker and poly-ε-lysine within an immiscible continuous phase.

In a preferred embodiment, X comprises a hydrocarbon group and comprises only hydrogen and carbon atoms, preferably 2 to 14, more preferably 3 to 12 and especially 5 to 8 carbon atoms. The hydrocarbon group may be linear or branched, preferably linear. The hydrocarbon group may be saturated or unsaturated, preferably saturated. Examples of preferred cross-linkers include di-carboxylic acids having 8 to 12 carbon atoms such as sebacic acid, dodecanedioic acid and azelaic acid.

The poly-ε-lysine may be derivatised or modified prior to cross-linking to allow other cross-linking chemistry familiar to those skilled in the art. The poly-ε-lysine may be cross-linked using an amino acid for example aspartic acid and glutamic acid. The cross-linked poly-ε-lysine in this case would only generate naturally occurring amino acids only upon degradation. Reaction with cystine for example would produce a polymer cross-linked in a similar way but in this instance the structure would contain a cysteine disulfide bridge, which again on degradation would generate naturally occurring amino acids.

Examples of preferred cross-linkers include glutamic acid, cystine, EDTA (ethylenediaminetetraacetic acid), adipic acid, dodecanedioic acid, synthetic peptides especially peptides based upon the structure of natural collagen, synthetic peptides containing the tripeptide sequence-Arg-Gly-Asp-, the cell binding peptide in fibrinogen and other natural proteins, naturally occurring polymers that contain multiple carboxylic acid groups including gelatin, alginic acid and crown ethers with multiple carboxylic acids especially suitable for use in metal chelation and chromatography.. Further suitable examples are shown below:

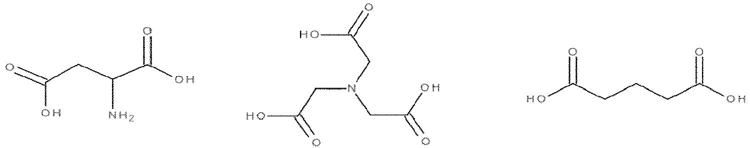

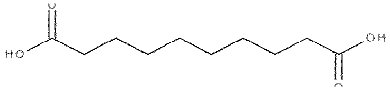

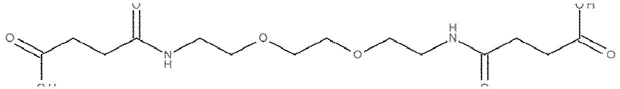

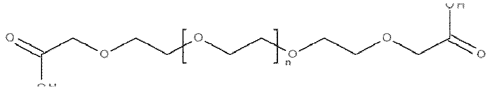

Whilst the polydispersity of the poly-ε-lysine is not critical, in a preferred embodiment the polydispersity of the poly-ε-lysine is suitably from 10 to 50 and preferably between 25-35 amino acids. A narrower polydispersity allows for more precise control over the ultimate properties of the cross-linked polymer support. Cross-linked poly-ε-lysine will have many applications for example in several modes of chromatography but may be of particular advantage for chiral separations due to the repeating L-chiral centers along the chain. The pendant α amino groups can be readily modified to incorporate other chromatographic binding sites or other chiral moieties that will impart a range of chiral selective properties on the support.

Suitably, the relative amount of poly-ε-lysine and cross-linker are selected such that the poly-ε-lysine is the principle component of the cross-linked poly-ε-lysine polymer. Where a fully cross-linked polymer is required, the relative amounts of poly-ε-lysine polymer and cross linker will be selected to provide stoichiometric molar equivalents as regards the alpha amine groups of the poly-ε-lysine and the cross-linking functional groups. Where amine functionality is desired, a corresponding lower amount of cross-linker is employed to provide the desired proportion of free amine groups. Suitably the cross-linked poly-ε-lysine polymer has from 0 to 95 % of its alpha amine groups as free amine groups. In a preferred embodiment in which a relatively large proportion of the amine groups for example 50 to 100%, have been reacted, the cross-linked poly-ε-lysine polymer will suitably exhibit relatively rigid characteristics. Where a minor proportion of the amine groups have been reacted to provide cross-linking, for example from 5 to up to 50 % of the amine groups, the polymer is suitably relatively soft or gel-like. Soft or gel-like polymers are especially useful in the synthesis of polypeptides, particularly long polypeptides.

In a preferred embodiment, the cross-linked poly-ε-lysine polymer comprises from greater than 0.001 to 20 mmol/g, 0.01 to 10mmol/g of cross-linked poly-ε-lysine polymer, and preferably from 0.1 to 8 mmol/g for example from 1 to 8 mmol/g especially for polypeptide synthesis.

In another embodiment, the cross-linked poly-ε-lysine polymer comprises from 0.01 to 0.3 mmol/g, particularly advantageous for the synthesis of nucleic acid sequences.

In a preferred embodiment, the cross-linker and poly-ε-lysine polymer are reacted in a solvent in which they are solvated for example water, which is dispersed within an immiscible continuous phase, for example dichloromethane. The concentration of the reactants in the solvent will affect the degree to which the cross-linked polymer shrinks on removal of the solvent and provides larger pores within the cross-linked polymer. Dimethylformamide may be employed as the solvent.

In a second aspect, the invention provides a particulate support comprising a cross-linked poly-ε-lysine polymer according to the invention.

Polymeric particles may typically be made by a dispersion or emulsion polymerization process in which a solution of monomers is dispersed in an immiscible solvent (continuous phase) prior to initiation of the polymerization. The polymer particles formed are typically then filtered, washed and classified to isolate the required particle size distribution.

In a preferred embodiment, the cross-linked polymer is formed on or around a particle, preferably a hollow particle. This provides a seeding effect and allows polymer particles of a narrow particle size distribution to be formed.

In a further preferred embodiment, the cross-linked polymer is formed on the surface of a particle. Preferably the particle is silica but may be another known polymer for example polystyrene and hydroxyl apatite. A coated silica particle is suitable for applications in high pressure liquid chromatography for example.

In a preferred embodiment, the cross-linked polymer is formed on or around nanoparticles. This provides a seeding effect and allows polymer particles of nanoparticle size to be formed. Such particles will have healthcare applications in drug delivery such as transfection for example.

The cross-linked poly-ε-lysine polymer may be reacted further to provide particular functionality for a given application. Suitably, the polymer is reacted with a compound having at least three functional groups, two for reacting with the polymer to provide cross-linking between two polymer chains and the other to provide free functionality for use in the desired application.

The cross-linked poly-ε-lysine support may be further functionalised, for example by conversion to a carboxylic acid using succinic acid as desired. By way of example, an amine functionalised support may be treated with N-hydroxysuccinimide and 1-Ethyl-3-[3-dimethylaminopropyl]carbodimide hydrochloride in preparation of an activated polymer for immobilising a protein, for example protein A.

The cross-linked polymer, preferably a particulate cross-linked polymer may also comprise a functional material supported by the polymer. Examples of suitable functional materials include a catalyst, an initiator species for peptide synthesis, a pharmaceutical active, an agrochemical active, a macromolecule, an enzyme, a nucleic acid sequence and a protein. Suitably, the cross-linked polymer is supported on a hollow particle. The cross-linked polymer may form a lattice or net around whole or part of the particle. The hollow particle suitably comprises functional groups, for example carboxylic acid groups which may react with free amine groups on the polymer and provide a covalent link between the cross-linked polymer and the particle.

The cross-linked poly-ε-lysine polymer or particulate support comprising the polymer invention is particularly useful in supporting precious metal catalysts, for example palladium catalysts. A particularly advantageous example is palladium acetate supported on cross-linked poly-ε-lysine functionalized to form a carboxylic acid.

The invention provides in a further aspect a method for producing a particulate support material comprising the steps of providing a particle, preferably a hollow particle, contacting the particle with a solution of poly-ε-lysine and cross-linker, effecting polymerisation of the poly-ε-lysine and cross-linker so as to form a polymer coating on the surface of the particle.

Suitably the polymerisation is initiated by a polymerisation initiation process known to those skilled in the art. In a preferred embodiment, hollow particles mixed with a solution of poly-ε-lysine and carboxyl functional cross-linker are added to a solvent which is immiscible with the poly-ε-lysine and cross-linker solvent and a carbodimide added to effect polymerisation. Where the poly-ε-lysine and cross-linker solution is aqueous, the solvent is for example toluene.

If preferred the polymer coating may be covalently linked to the particle either during the polymerization or subsequent to the polymerization. Alternatively, one or more of the poly-ε-lysine and cross-linker can be covalently linked to the particle surface prior to initiation of the polymerization. Preferably the particle is hollow.

The particulate support of the invention may be used in any chemical or physical process in which a solid support is used.

The support may be employed in applications involving electro-conducting and light emitting polymers. The support containing light emitting polymers may be arranged on display panels.

The particulate support is particularly useful for solid phase synthesis of an organic species, particularly macromolecules. In a preferred embodiment the particulate support may be employed in the synthesis of peptides, oligonucleotides or oligosaccharides.

The invention further provides for the use of a particulate support according to the invention as a solid phase in a chromatography process.

The particulate support of the invention is also useful for solid phase extraction to remove species from a liquor which is contacted with the support, whether in batch form or as a flow over the support, for example ion extraction and ion exchange.

The support of the invention may be used to immobilize species including antibodies, oligonucleotides, enzymes or fluors and may be positioned in an array, with each support assaying a different component of a solution. Particles having ligands covalently attached to their surface, or via polymers bound to the surface may be employed as 'wells'. Specific binding of a target ligand such as antigen or complimentary DNA or RNA sequence may then be detected using established methods.

The particulate support of the invention also may be employed to immobilize a biocatalyst or whole cells for use in biocatalysis. Biocatalysts are often used in columns or in systems with filter plates for separation of the solid phase from the mixture under extraction.

The particulate support of the invention is especially useful in immobilizing species including solid phase reagents, metal and other catalysts, bio-catalysts, enzymes, proteins, antibodies including polyclonal and monoclonal antibodies, whole cells and polymers. The invention is particularly advantageous in supporting enzymes, for example the lipase Cal B. Lipase Cal B may be employed in a transesterification process.

The invention further provides for a method of enzymatic manufacture of biodiesel using a support of the invention.

The present invention is also especially useful in the immobilisation of affinity ligands such as Protein A. Protein A is suitably used in the purification of monoclonal antibodies.

In a further application, the particulate support of the invention may also be used in chemocatalysis, for example by immobilizing transition metal catalysts and ligands.

In yet a further application, the present invention may be used in cell culture. Mass culture of animal cell lines is fundamental to the manufacture of viral vaccines and many products of biotechnology. Biological products produced by recombinant DNA technology in animal cell cultures include enzymes, synthetic hormones, immunobiologicals (monoclonal antibodies, interleukins, lymphokines) and anticancer agents. Many simpler proteins can be produced using rDNA in bacterial cultures; more complex proteins that are glycosylated (carbohydrate-modified) currently must be made in animal cells. An important example of such a complex protein is the hormone erythropoietin. The cost of growing mammalian cell cultures is high, so companies are constantly looking to improve techniques.

Cells can be grown in suspension or as adherent cultures. However, adherent cells require a surface, which may be coated with extracellular matrix components to increase adhesion properties and provide other signals needed for growth and differentiation. Generally cells derived from solid tissues are adherent. Organotypic culture involves growing cells in a three-dimensional environment as opposed to two-dimensional culture dishes. This 3D culture system is biochemically and physiologically more similar to in vivo tissue, but is technically challenging to maintain because of many factors (e.g. diffusion). Gelatin is hydrolyzed collagen where inter and intra chain amide bonds have been hydrolyzed chemically to form soluble peptide chains. Collagen is an ideal and natural environment for cells to adhere and differentiate. The poly-ε-lysine may also be co-polymerized with other proteins, for example gelatin to form a collagen like environment.

In a further aspect, the invention provides for the use of a particulate, macroporous or microporous support or coating according to the invention to culture cells preferably on the surface of the support or coating. Suitably, stem cells may be cultured on the particulate support of the invention to reduce uncontrolled differentiation and to control desired differentiation.

In an especially preferred embodiment, the invention is beneficial for use in wound care. Chronic wounds are exacerbated by metallino-proteases which can be rendered inactive by polymer particles that chelate metal ions required by the enzyme. The cross-linked poly -ε-lysine, preferably capped with metal chelating species are suitable for use in this application. The invention provides for the use of cross-linked poly -ε-lysine or a particulate support comprising cross-linked poly -ε-lysine as a wound treatment product or component thereof. The wound treatment product may comprise a flexible article but preferably comprises a self-supporting article. The wound treatment product suitably comprises a polymer or particulate support according to the invention and a component or a composition for treating a wound and/or a therapeutic agent.

The invention is particular useful in medical diagnostic tests such as immunoassay. Accordingly the invention further provides medical diagnostics for detecting the presence of a compound comprising a polymer according to the invention and a functional material such as an enzyme, for example horseradish peroxidase, supported by the polymer in the support for selectively reacting with or binding to the compound to be detected.

Many medical diagnostics rely upon solid supports to immobilize various diagnostic ligands. The polymer support of the present invention may be used in a medical diagnostic procedure where physical separation of the solid phase through a liquid phase.

In a further application, the polymer support may be used as an absorbent. The polymer support may be used to absorb household spillages, for example tea, coffee and wine, or may be used in larger-scale applications for example, to absorb oil from spillages. The absorbent support may be used to absorb the spillage and then left to biodegrade or, in the case of oil spillage in a body of water, effectively trap the oil and retain the oil in a buoyant mass for collection and disposal. Advantageously, the cross-linked poly-ε-lysine is biodegradable facilitating disposal with reduced environmental impact.

The polymer support of the invention may be used as a biodegradable carrier to carry a compound which is to be released over a period of time, for example a pharmaceutical or agrochemical compound or composition. This use provides a means of tailoring a dosage regime of the compound according to the loading of the compound in the support. In the case of a pharmaceutical, this may be advantageous in assisting the correct dosage of an active, for example with continuous slow release rather than requiring a patient to take periodic large doses, for example in chemotherapy.

The invention is illustrated by reference to the accompanying drawings in which:

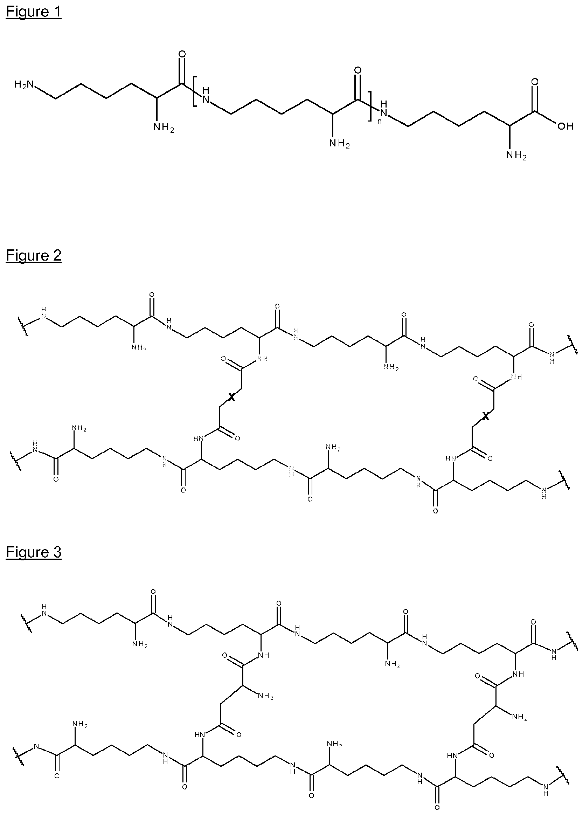

Figure 1 shows a diagrammatic representation of poly-ε-lysine.
Figure 2 shows a diagrammatic representation of poly-ε-lysine cross-linked with a bifunctional carboxylic acid.
Figure 3 shows a diagrammatic representation of poly-ε-lysine cross-linked with aspartic acid as example.
Figure 4 shows a diagrammatic representation of poly-ε-lysine cross-linked with cystine.
Figure 5 shows a diagrammatic representation of poly-ε-lysine cross-linked with nitrilotriacetic acid.
Figures 6a and 6b show photographs of the Pd²⁺ and Pd⁰ chelated microspheres.
Figure 7 shows a microscope photograph of the particles.
Figure 8 shows an HPLC test chromatogram.

The invention is illustrated by the following non-limiting examples.

### Example 1 - Preparation of particles having amine active surface

### 1. Particle surface hydrolysis (to produce -COOH)

Hollow polymer particles (500cm³, Akzo Nobel - Expancel, grade 920 DEX80) were placed in a 1dm³ Polypropylene bottle and covered with sodium hydroxide solution (100g in 500cm³ 4:1v/v water / methanol). The mixture was left on a roller at room temperature overnight.

The particles were washed in a separating funnel with water (5 x 500cm³), aqueous hydrochloric acid (1mol/dm³, 5 x 500cm³) then water (5 x 500cm³). The particles were then washed with methanol (5 x 500cm³) and air dried. Particles having free carboxylic acid groups on the surface of the [particle were created.

### 2. Coating of particles with cross-linked poly-ε-lysine

DEX80 particles prepared above (50cm³, -COOH functional) were placed in a round bottomed flask and suspended in a solution (100cm³) of poly-ε-lysine (10g, 51.5mmol of - NH₂), nitrilotriacetic acid (2.64g, 41.5mmol COOH) and N-methylmorpholine (4.2g, 41.5mmol). This slurry was dispersed with rapid stirring in dichloromethane (DCM) (250cm³) containing SPAN 80 (2%w/v). A solution of 1-(3-dimethylaminopropyl)-3-ethylcarbodiimide hydrochloride (EDCI) (23.9g, 125mmol) in DCM (250cm³) was added and the polymerisation allowed to proceed for 2h.

The DCM layer was removed by decantation and the particles were washed on a 75µm stainless steel sieve with copious amounts of tap water. The particles were transferred to a glass sinter and washed with water (5 x 500cm³) then washed with methanol (5 x 500cm³) and air dried (yield 5.8g).

The amine loading of the cross-linked poly-ε-lysine was determined by coupling Fmoc-Gly-OH to the polymer followed by Fmoc assay. The loading was 0.9mmol/g (theoretical maximum 1mmol/g).

### 3. Coating of particles with cross-linked poly-ε-lysine

DEX80 particles prepared above (50cm³, -COOH functional) were placed in a round bottomed flask and suspended in a solution (100cm³) of poly-ε-lysine (10g, 51.5mmol of - NH₂), HOOCCH₂CH₂CONHCH₂CH₂OCH₂CH₂OCH₂CH₂OCH₂CH₂NHCO CH₂CH₂COOH (4.99g, 26.7mmol COOH) and N-methylmorpholine (2.7g, 26.7mmol). This slurry was dispersed with rapid stirring in toluene (250cm³) containing SPAN 80 (5%w/v). A solution of EDCI (10g, 52mmol) in DCM (250cm³) was added and the polymerisation allowed to proceed for 1.5h.

The organic layer was removed by decantation and the particles were washed on a 75µm stainless steel sieve with acetone (∼1dm³) and copious amounts of tap water. The particles were transferred to a glass sinter and washed with water (5 x 500cm³) then washed with methanol (5 x 500cm³) and air dried (yield 11.5g, ∼90% of theory).

The amine loading of the cross-linked poly-ε-lysine was determined by coupling Fmoc-Gly-OH to the polymer followed by Fmoc assay. The loading was 0.84mmol/g (theory 1.7mmol/g).

### Example 2 - Synthesis of the peptide ACP(64-75)

### 1. Coupling of linkage agent

4-Hydroxymethylphenoxyacetic acid (HMPA) (0.5g, 3mmol) was coupled to the cross-linked poly-ε-lysine prepared in Example 1.2 above (1.1g, 0.9mmol) using DIC (0.5g, 4mmol) and HOBt (0.81g, 6mmol) in N,N-dimethylformamide (DMF) (10cm³).

The coupling reaction was complete by Ninhydrin assay within 90min. The polymer support was washed on a glass sinter with aliquots of DMF (10x10cm³).

### 2. Coupling of Fmoc-Gly-OH

Fmoc-Gly-OH (0.89g, 3mmol) was coupled to the HMPA-polymer prepared above using DIC (0.5g, 4mmol) and DMAP (12mg, 0.1mmol) in N,N-dimethylformamide (DMF) (10cm³) for 60min.

The coupling was repeated and the polymer was washed on a glass sinter with aliquots of DMF (10x10cm³).

Piperidine/DMF (20cm³, 20%v/v) was added and the reaction was allowed to stand for 3 minutes. A second treatment with Piperidine/DMF (20cm³, 20%v/v) for 7minutes was carried out and the polymer support washed with DMF (10x10cm³).

### 3. Coupling of Fmoc-Asn(Trt)-OH

Fmoc-Asn(Trt)-OH (1.492g, 2.5mmol) and 2-(1H-benzotriazol-1-yl)-N, N,N',N'-tetramethyluronium tetrafluoroborate (TBTU) (0.763g, 2.38mmol) were dissolved in DMF (5cm³). 4-Methylmorpholine (NMM) (0.412cm³, 3.7mmol) was added and the mixture pre-activated for 2-3 minutes before adding to the above H-Gly-HMPA-polymer.

The coupling reaction was coupled to completion as determined by Ninhydrin assay. The polymer support was washed with aliquots (10x10cm³) of DMF.

Piperidine/DMF (20cm³, 20%v/v) was added to the mixture. The reaction was allowed to stand for 3 minutes. A second treatment with Piperidine/DMF (20cm³, 20%v/v) for 7minutes was carried out and the polymer support washed with DMF (10x10cm³).

### 4. Coupling of Fmoc-Ile-OH

Fmoc-Ile-OH (0.884g, 2.5mmol) and TBTU (0.763g, 2.38mmol) were dissolved in DMF (5cm³). NMM (0.412cm³, 3.7mmol) was added and the mixture pre-activated for 2-3 minutes before adding to the above peptide-HMPA-polymer.

The coupling reaction was coupled to completion as determined by Ninhydrin assay. The polymer support was washed with aliquots (10x10cm³) of DMF.

Piperidine/DMF (20cm³, 20%v/v) was added to the mixture. The reaction was allowed to stand for 3 minutes. A second treatment with Piperidine/DMF (20cm³, 20%v/v) for 7minutes was carried out and the polymer support washed with DMF (10x10cm³).

### 5. Coupling of Fmoc-Tyr(tBu)-OH

Fmoc-Tyr(tBu)-OH (1.149g, 2.5mmol) and TBTU (0.763g, 2.38mmol) were dissolved in DMF (5cm³). NMM (0.412cm³, 3.7mmol) was added and the mixture pre-activated for 2-3 minutes before adding to the above peptide-HMPA-polymer.

The coupling reaction was coupled to completion as determined by Ninhydrin assay. The polymer support was washed with aliquots (10x10cm³) of DMF.

Piperidine/DMF (20cm³, 20%v/v) was added the mixture. The reaction was allowed to stand for 3 minutes. A second treatment with Piperidine/DMF (20cm³, 20%v/v) for 7minutes was carried out and the polymer support washed with DMF (10x10cm³).

### 6. Coupling of Fmoc-Asp(OtBu)-OH

Fmoc-Asp(OtBu)-OH (1.029g, 2.5mmol) and TBTU (0.763g, 2.38mmol) were dissolved in DMF (5cm³). NMM (0.412cm³, 3.7mmol) was added and the mixture pre-activated for 2-3 minutes before adding to the above peptide-HMPA-polymer.

The coupling reaction was coupled to completion as determined by Ninhydrin assay. The polymer support was washed with aliquots (10x10cm³) of DMF.

Piperidine/DMF (20cm³, 20%v/v) was added to the mixture. The reaction was allowed to stand for 3 minutes. A second treatment with Piperidine/DMF (20cm³, 20%v/v) for 7minutes was carried out and the polymer support washed with DMF (10x10cm³).

### 7. Coupling of Fmoc-Ile-OH

Fmoc-Ile-OH (0.884g, 2.5mmol) and TBTU (0.763g, 2.38mmol) were dissolved in DMF (5cm³). NMM (0.412cm³, 3.7mmol) was added and the mixture pre-activated for 2-3 minutes before adding to the above peptide-HMPA-polymer.

The coupling reaction was coupled to completion as determined by Ninhydrin assay. The polymer support was washed with aliquots (10x10cm³) of DMF.

Piperidine/DMF (20cm³, 20%v/v) was added to the mixture. The reaction was allowed to stand for 3 minutes. A second treatment with Piperidine/DMF (20cm³, 20%v/v) for 7minutes was carried out and the polymer support washed with DMF (10x10cm³).

### 8. Coupling of Fmoc-Ala-OH

Fmoc-Ala-OH (0.823g, 2.5mmol) and TBTU (0.763g, 2.38mmol) were dissolved in DMF (5cm³). NMM (0.412cm³, 3.7mmol) was added and the mixture pre-activated for 2-3 minutes before adding to the above peptide-HMPA-polymer.

The coupling reaction was coupled to completion as determined by Ninhydrin assay. The polymer support was washed with aliquots (10x10cm³) of DMF.

Piperidine/DMF (20cm³, 20%v/v) was added to the mixture. The reaction was allowed to stand for 3 minutes. A second treatment with Piperidine/DMF (20cm³, 20%v/v) for 7minutes was carried out and the polymer support washed with DMF (10x10cm³).

### 9. Coupling of Fmoc-Ala-OH

Fmoc-Ala-OH (0.823g, 2.5mmol) and TBTU (0.763g, 2.38mmol) were dissolved in DMF (5cm³). NMM (0.412cm³, 3.7mmol) was added and the mixture pre-activated for 2-3 minutes before adding to the above peptide-HMPA-polymer.

The coupling reaction was coupled to completion as determined by Ninhydrin assay. The polymer support was washed with aliquots (10x10cm³) of DMF.

Piperidine/DMF (20cm³, 20%v/v) was added to the mixture. The reaction was allowed to stand for 3 minutes. A second treatment with Piperidine/DMF (20cm³, 20%v/v) for 7 minutes was carried out and the polymer support washed with DMF (10x10cm³).

### 10. Coupling of Fmoc-Gln(Trt)-OH

Fmoc-Gln(Trt)-OH (1.607g, 2.5mmol) and TBTU (0.763g, 2.38mmol) were dissolved in DMF (5cm³). 4-Methylmorpholine (NMM) (0.412cm³, 3.7mmol) was added and the mixture pre-activated for 2-3 minutes before adding to the above peptide-HMPA-polymer.

The coupling reaction was coupled to completion as determined by Ninhydrin assay. The polymer support was washed with aliquots (10x10cm³) of DMF.

Piperidine/DMF (20cm³, 20%v/v) was added to the mixture. The reaction was allowed to stand for 3 minutes. A second treatment with Piperidine/DMF (20cm³, 20%v/v) for 7minutes was carried out and the polymer support washed with DMF (10x10cm³).

### 11. Coupling of Fmoc-Val-OH

Fmoc-Val-OH (0.848g, 2.5mmol) and TBTU (0.763g, 2.38mmol) were dissolved in DMF (5cm³). NMM (0.412cm³, 3.7mmol) was added and the mixture pre-activated for 2-3 minutes before adding to the above peptide-HMPA-polymer.

The coupling reaction was coupled to completion as determined by Ninhydrin assay. The polymer support was washed with aliquots (10x10cm³) of DMF.

Piperidine/DMF (20cm³, 20%v/v) was added to the mixture. The reaction was allowed to stand for 3 minutes. A second treatment with Piperidine/DMF (20cm³, 20%v/v) for 7minutes was carried out and the polymer support washed with DMF (10x10cm³) and diethyl ether (5x10cm³) before air drying (yield 1.93g, 86% of theory).

### 12. Peptide cleavage

Trifluoroacetic acid (TFA) (20cm³) containing water (2.5%v/v) and triisopropylsilane (TIPS) (2.5%v/v) was added to the peptide-HMPA-polymer prepared above (0.93g). The mixture was left to cleave for 2 hours.

The polymer support was washed with TFA (5x15cm³) in a separating funnel. The combined TFA cleavage solutions and washes were reduced to an oil on a rotary evaporator. The oil was triturated with diethyl ether to form a white solid. The ether removed by decantation and the peptide air dried overnight (yield 0.4g).

The peptide was shown to contain one major component by reversed phase HPLC and co-eluted with a sample of ACP(64-75) prepared on a traditional polymer support. The peptide prepared on this cross-linked poly-ε-lysine was a slightly higher quality compared to the ACP(64-75) prepared on the traditional polymer support.

### Example 3 - Synthesis of the peptide ACP(64-75)

The procedure of Example 2 was repeated using the cross-linked polymer. Preparation of the ACP(64-75) as described above in Example 2 on the cross-linked poly-ε-lysine prepared in Example 1.3 produced a peptide of similar quality even when all couplings were limited to 1 hour.

### Example 4 - Immobilisation of Protein A on cross-linked poly-ε-lysine Coupling of rProtein A to cross-linked poly-ε-lysine

To convert the amines on the polymer support to carboxylic acid groups a cross-linked poly-ε-lysine with 0.1mmol/g amine loading (0.2g) was treated with glutaric anhydride (2g) in water (5cm³) overnight (resultant polymer was ninhydrin negative). The polymer support was washed on a glass sinter with water (5x10cm³). Morpholinoethane sulfonic acid (MES) buffer (25mmol/dm³, pH 5.0, 3x20cm³) was used to wash the polymer support prior to protein coupling.

N-hydroxysuccinimide (1g) was dissolved in cold MES buffer (25mmol/dm³, pH 5.0, 2.5cm³) and this solution added to the polymer support. EDCI (1g) was dissolved in MES buffer (25mmol/dm³, pH 5.0, 2.5cm³) and this solution added to the mixture. The polymer support was mixed for 30 minutes on a bottle roller to activate. The polymer support was washed with MES buffer (25mmol/dm³, pH 5.0, 5x10cm³) and immediately, a solution of rProtein A (5cm³, 4mg/cm³ in 25mmol/dm³ MES, pH 5.0) was added to the mixture and coupling allowed to proceed over a weekend at room temperature with mixing on a bottle roller. The polymer support was drained on a glass sinter and washed with Trizma-HCl (30cm³ pH 7.4) to block any remaining N-hydroxysuccinimide esters on the polymer. The polymer support was washed with water (10x10cm³) and stored as a slurry in water.

The Protein A based media was tested to determine whether it retained Human IgG using a packed chromatography column under standard conditions known to those skilled in the art using Human IgG. The column was shown to retain Human IgG as expected.

### Example 5 - Preparation of spherical particles cross-linked with sebacic acid

Poly-ε-lysine (42.88g, 250mmol amine content), sebacic acid (12.64g, 125mmol carboxyl content) and NaOH (5.5g, 137.5mmol) were dissolved in water (208cm³) then dispersed with rapid stirring in dichloromethane (DCM) (1.25dm³) containing 1.5%m/v SPAN 80.

EDCI (36g, 187.5mmol) was dissolved in DCM (250cm³) and added to the above dispersion. The polymerisation was allowed to proceed for 90 minutes. The DCM was removed by filtration and the polymer microspheres washed with DCM (3x500cm³) to remove residual Span 80. The polymer was then washed thoroughly with water, neutralised with NaOH solution (1mol/dm³) then washed again with water followed by MeOH (3x500cm³), DCM (3x500cm³) then Et₂O (2x250cm³). The polymer microspheres were air dried overnight (yield 34g).

### Example 6 - Immobilisation of the lipase Cal B

A sample of the polymer microspheres (2g) prepared in Example 5 was measured into a polypropylene bottle. Glutaric anhydride (6g) was dissolved in a solution of methanol (20cm³) and N-methylmorpholine (6cm³), added to the polymer and placed on the roller machine for 24 h.

After 24 h the polymer was washed thoroughly with water. A saturated solution of N-hydroxysuccinimide (3.2g) and ECDI (5.3g) in water (20cm³) was added to the spheres in a polypropylene bottle and placed back on the roller for 1 h in order to activate carboxyl. After 1 h a sample (50 mg) was removed to act as the negative control in the BCA (bicinchoninic acid) assay that followed. The rest of the polymer was washed thoroughly with water then added to a bottle containing 10% Cal B enzyme solution (40cm³) before being placed on the roller machine for 24 h to allow the enzyme to couple to the activated carboxylic acid. After 24 h the polymer was washed thoroughly with water to remove any uncoupled enzyme before being washed in a 5%v/v solution of ethanolamine to cap off any remaining free carboxyl groups thereby preventing any further reactions from taking place.

A BCA assay was carried out using a Pierce® BCA assay kit (No. 23225) to determine the Cal B loading on the polymer. The loading was determined to be 218mg Cal B per g of polymer.

The activity of the immobilized enzyme was shown to be equivalent to the free enzyme in solution confirming that the immobilized enzyme could be used in transesterification reactions including for example, the enzymatic production of biodiesel.

### Example 7 - Preparation of particles cross-linked with nitrilotriacetic acid (NTA)

Poly-ε-lysine (40g, 206mmol amine content), NTA (10.96g, 172mmol carboxyl content) and N-methylmorpholine (17.37g, 172mmol) were dissolved in water (140cm³) then dispersed with rapid stirring in dichloromethane (DCM) (750cm³) containing 2%m/v SPAN 80.

EDCI (49.5g, 258mmol) was dissolved in DCM (400cm³) and added to the above dispersion. The polymerisation was allowed to proceed for 90 minutes. The DCM was removed by filtration and the polymer microspheres washed with DCM (3x500cm³) to remove residual Span 80. The polymer was then washed thoroughly with water, neutralised with NaOH solution (1mol/dm³) then washed again with water followed by MeOH (3x500cm³), DCM (3x500cm³) then Et₂O (2x250cm³). The polymer microspheres were air dried overnight (yield 47g).

### Example 8 - Preparation of particles cross-linked with NTA capped with NTA for nickel chelation and subsequent immobilised metal affinity chromatography (IMAC)

A portion of the polymer microspheres (10g, 10mmol) prepared in Example 7 above was swollen in water in a polypropylene bottle. A solution of EDCI (19.17g, 100mmol) and NTA (19.1g, 100mmol) in water (∼100cm3) was prepared and added to the swollen microspheres. The mixture was left on a roller mill overnight to complete the capping with NTA.

The polymer was washed thoroughly with water followed by MeOH (3x100cm³), DCM (3x100cm³) then Et₂O (2x50cm³) before air drying. This material was now suitable for chelation with Nickel for subsequent use in IMAC.

This IMAC polymer was confirmed to be suitable for His-tagged protein purification by techniques known to those skilled in the art.

### Example 9 - Preparation of particles cross-linked with ethyenediaminetetraacetic acid (EDTA)

Poly-ε-lysine (42.88g, 250mmol amine content), EDTA (9.13g, 125mmol carboxyl content) were dissolved in water (200cm³) then dispersed with rapid stirring in dichloromethane (DCM) (750cm³) containing 2%m/v SPAN 80.

EDCI (36g, 187.5mmol) was dissolved in DCM (250cm³) and added to the above dispersion. The polymerisation was allowed to proceed for 90 minutes. The DCM was removed by filtration and the polymer microspheres washed with DCM (3x500cm³) to remove residual Span 80. The polymer was then washed thoroughly with water, neutralised with NaOH solution (1mol/dm³) then washed again with water followed by MeOH (3x500cm³), DCM (3x500cm³) then Et₂O (2x250cm³). The polymer microspheres were air dried overnight (yield 27g).

### Example 10 - Preparation of particles cross-linked with EDTA and capped with EDTA for subsequent metal chelation

A portion of the polymer microspheres (10g, 10mmol) prepared in Example 9 above was swollen in water in a polypropylene bottle. A solution of EDCI (19.17g, 100mmol) and EDTA (29.2g, 100mmol) in water (∼100cm3) was prepared and added to the swollen microspheres. The mixture was left on a roller mill overnight to complete the capping with EDTA.

The polymer was washed thoroughly with water followed by MeOH (3x100cm3), DCM (3x100cm3) then Et2O (2x50cm3) before air drying. This material was now suitable for metal chelation.

To confirm the metal chelation properties, 1g of this polymer was added to a solution of palladium acetate (100mg) in acetic acid (20cm³). After 2h at room temperature the orange colour of the palladium acetate solution faded and the colour was transferred to the polymer microspheres. The spheres were washed with methanol and left in methanol to convert the chelated Pd²⁺ to Pd°.

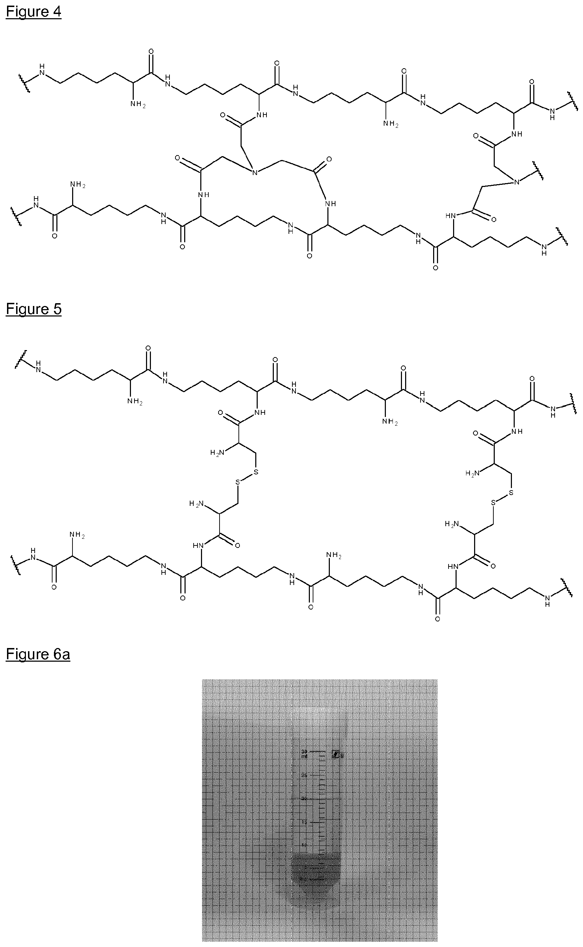

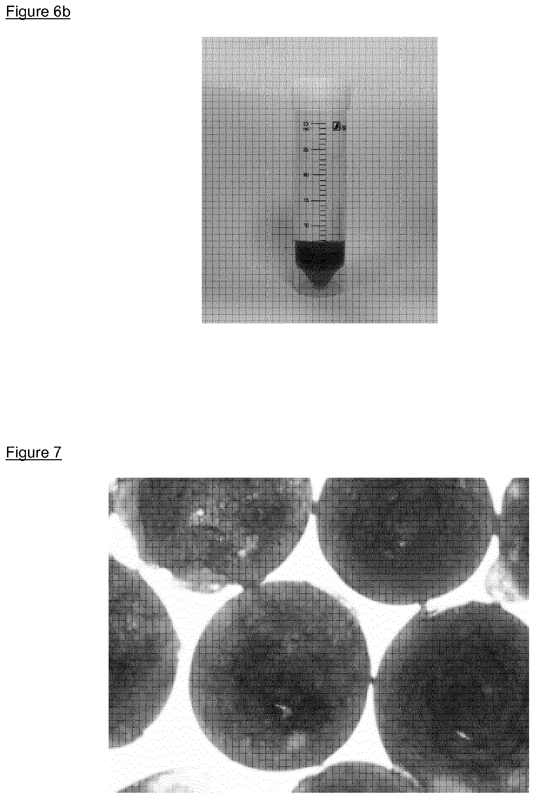

Photographs of the Pd²⁺ and Pd° chelated microspheres are shown in Figures 6a and 6b.

### Example 11 - Preparation of buoyant superparamagnetic particles

DEX80 hollow particles (50cm³) were placed in a round bottomed flask and suspended in a solution (100cm³) of poly-ε-lysine (10g, 51.5mmol of -NH₂), sebacic acid (2.6g, 25.75mmol COOH) and N-methylmorpholine (2.6g, 25.75mmol). Superparamagnetic iron oxide nanoparticles (1 g, 50nm particle size) were added and the slurry was dispersed with rapid stirring in dichloromethane (DCM) (250cm³) containing SPAN 80 (2%w/v). A solution of EDCI (23.9g, 125mmol) in DCM (250cm³) was added and the polymerisation allowed to proceed for 2h.

The DCM layer was removed by filtration and the particles were washed on a 500µm stainless steel sieve with copious amounts of tap water. The particles were transferred to a glass sinter and washed with water (5 x 500cm³) and stored as a slurry in water.

A microscope photograph of the particles is shown in Figure 7.

### Example 12 - Preparation of superparamagnetic nanoparticles

Superparamagnetic iron oxide nanoparticles (3g, 50nm particle size) were transferred to a thick walled glass tube. A saturated solution of citric acid in water was added and the mixture sonicated for 30 minutes at 40°C. The nanoparticles were washed with water (10x30cm³) using the magnetic properties to retain the particles during decantation.

Poly-ε-lysine (2g) was dissolved in water (5cm³) and added to the citric acid chelated nanoparticles. The mixture sonicated for 30 minutes at room temperature then EDCI (2g) dissolved in water (2cm³) was added. The mixture sonicated for a further 30 minutes at 40°C then left at room temperature overnight.

The nanoparticles were washed with water (10x30cm³), MeOH (5x30cm³) and Et₂O (10cm³) using the magnetic properties to retain the particles during decantation. The nanoparticles were air dried (yield 3.9g).

The resultant citric acid cross-linked, poly-ε-lysine coated nanoparticles were ninhydrin positive confirming the presence of amine coating. The amine content of the nanoparticles was determined by coupling Fmoc-Leu-OH to the polymer followed by determination of the Fmoc content. The amine loading was determined to be 66µmol/g.

### Example 13 - Preparation of silica particles coated with poly-ε-lysine and capped with palmitic acid.

Silica particles (10g, Kromasil, 10um, 1000Å pore) were suspended in a solution of aminopropyltrimethoxysilane in MeOH/water (50cm³, 1%w/v, 10% water), mixed for 5-10 minutes then washed with MeOH and DMF before transferring to a polypropylene bottle. DMF (50cm³) was added followed by glutaric anhydride (5g) and N-methylmorpholine (10cm³). The mixture was left on a roller mixer overnight.

The particles were washed thoroughly with DMF then re-suspended in DMF (50cm³). N-hydroxysuccinimide (1.72g) and diisopropylcarbodiimide (DIC) (3.37cm³) were added and the mixture rolled for 5h. The particles were again washed thoroughly with DMF.

Poly-ε-lysine (1.57g) was dissolved in water (25cm³) and added to the DMF damp silica particles. N-methylmorpholine (0.44cm³) was added and the mixture rolled overnight.

The particles were washed thoroughly with water, MeOH and DMF. Palmitic acid (1.2g), N-hydroxysuccinimide (0.52g) and DIC (1.1cm³) were dissolved in DMF (50cm³) and added to the DMF damp silica particles. N-methylmorpholine (0.74cm³) was added and the mixture rolled overnight. The palmitic acid coupling was repeated and the particles washed thoroughly with DMF, water and MeOH before drying in air.

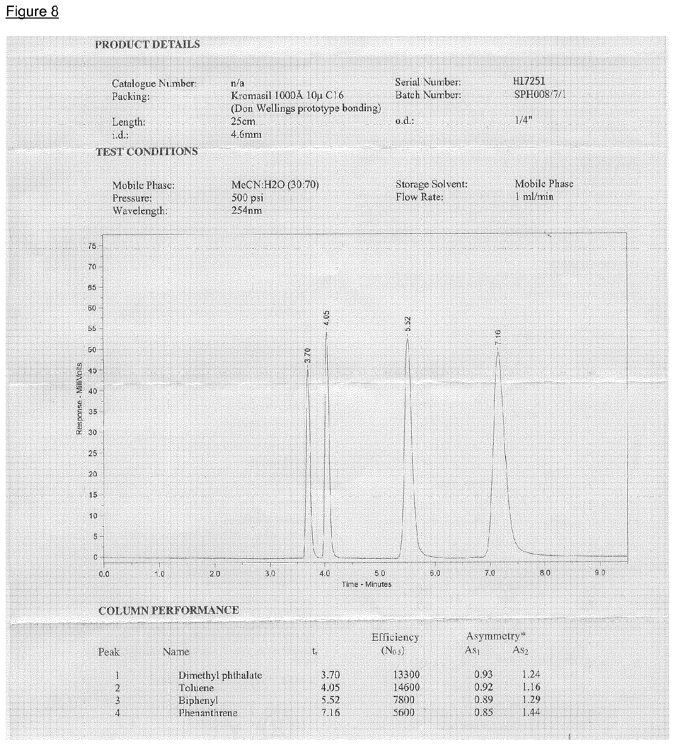

These reversed phase chromatography particles were packed in and HPLC column and tested to confirm the chromatographic properties. The HPLC test chromatogram is shown in Figure 8.

## Claims

1. A cross-linked poly-ε-lysine polymer comprising poly-ε-lysine and a cross linker linked by amide bonds wherein the cross linker comprises two or more carboxylic acid groups and an aliphatic chain linking the two or more groups adapted to react with an alpha carbon amine of poly-ε-lysine.

2. A polymer according to claim 1 which is insoluble in water and other solvents.

3. A polymer according to any one of claim 1 or claim 2 in particulate form.

4. A particulate support comprising a cross-linked poly-ε-lysine polymer according to any one of the preceding claims.

5. A particulate support according to claim 4 wherein the cross-linked poly-ε-lysine support is a spherical, ellipsoidal or irregular particle.

6. A particulate support according to claim 4 or claim 5 wherein the cross-linked poly-ε-lysine support is used to coat a particle directly or indirectly.

7. A particulate support according to any one of claims 4 to 6 wherein the cross-linked poly-ε-lysine support is used to coat and is bound covalently to a particle.

8. A particulate support according to any one of claims 4 to 7 wherein the cross-linked poly-ε-lysine is used to coat an organic polymer particle.

9. A particulate support according to any one of claims 4 to 7 wherein the cross-linked poly-ε-lysine is used to coat an inorganic polymer particle.

10. A particulate cross-linked poly-ε-lysine based support according to any one of claims 4 to 9 wherein the cross-linked poly-ε-lysine is functionalised to provide a material comprising a catalyst, an initiator species for peptide synthesis, an initiator species for oligonucleotide synthesis , an initiator species for solid phase organic synthesis, a pharmaceutical active, an agrochemical active, a surface for chromatographic separation, a species to promote cell culture or differentiation, a protein or other biological macromolecule.

11. A wound treatment product comprising a polymer according to any one of claims 1 to 3 or particulate support according to any one of claims 4 to 10.

12. A wound treatment product according to claim 11 which further comprises a component or a composition for treating a wound and/or a therapeutic agent.

13. A medical diagnostic comprising a particulate support according to any one of claims 4 to 10 and comprising a functional material bound or retained by the support.

14. A medical diagnostic according to claim 13 wherein the functional material comprises an enzyme supported by the polymer.

15. Use of a particulate support according to any one of claims 4 to 10 in a process selected from solid phase synthesis of peptides, oligonucleotides, oligosaccharides; solid phase extraction; solid phase organic chemistry; immobilisation of a species selected from solid phase reagents, metal and other catalysts, bio-catalysts, enzymes, proteins, antibodies including polyclonal and monoclonal antibodies, whole cells and polymers; cell culturing; preparation of a stationary phase for chromatographic separation; or for use as an absorbent or as a biodegradable carrier for a controlled release product.

## Patentansprüche

1. Vernetztes Poly-ε-Lysin-Polymer, umfassend Poly-ε-Lysin und einen Vernetzer, verknüpft durch Amidbindungen, wobei der Vernetzer umfasst: zwei oder mehr Carbonsäuregruppen und eine aliphatische Kette, die die zwei oder mehr Gruppen verbindet, und geeignet ist, um mit einer Aminogruppe am α-Kohlenstoffatom von Poly-ε-Lysin zu reagieren.

2. Polymer nach Anspruch 1, das unlöslich in Wasser und anderen Lösungsmitteln ist.

3. Polymer nach einem von Anspruch 1 oder Anspruch 2 in Teilchenform.

4. Teilchenförmiges Trägermaterial, umfassend ein vernetztes Poly-ε-Lysin-Polymer nach einem der vorangehenden Ansprüche.

5. Teilchenförmiges Trägermaterial nach Anspruch 4, wobei das vernetzte Poly-ε-Lysin - Trägermaterial ein kugelförmiges, ellipsoides oder unregelmäßiges Teilchen ist.

6. Teilchenförmiges Trägermaterial nach Anspruch 4 oder Anspruch 5, wobei das vernetzte Poly-ε-Lysin - Trägermaterial verwendet wird, um ein Teilchen direkt oder indirekt zu beschichten.

7. Teilchenförmiges Trägermaterial nach einem der Ansprüche 4 bis 6, wobei das vernetzte Poly-ε-Lysin - Trägermaterial verwendet wird, um zu beschichten und kovalent an ein Teilchen gebunden wird.

8. Teilchenförmiges Trägermaterial nach einem der Ansprüche 4 bis 7, wobei das vernetzte Poly-ε-Lysin verwendet wird, um organische Polymerteilchen zu beschichten.

9. Teilchenförmiges Trägermaterial nach einem der Ansprüche 4 bis 7, wobei das vernetzte Poly-ε-Lysin verwendet wird, um anorganische Polymerteilchen zu beschichten.

10. Teilchenförmiges Trägermaterial auf Basis von vernetztem Poly-ε-Lysin nach einem der Ansprüche 4 bis 9, wobei das vernetzte Poly-ε-Lysin funktionalisiert ist zum Bereitstellen eines Materials, umfassend einen Katalysator, eine Initiatorspezies für die Peptidsynthese, eine Initiatorspezies für die Oligonucleotidsynthese, eine Initiatorspezies für die organische Festphasensynthese, einen pharmazeutischen Wirkstoff, einen agrochemischen Wirkstoff, eine Oberfläche für die chromatographische Trennung, eine Spezies zur Förderung von Zellkultur oder Differenzierung, ein Protein oder ein anderes biologisches Makromolekül.

11. Wundbehandlungsprodukt, umfassend ein Polymer nach einem der Ansprüche 1 bis 3 oder ein teilchenförmiges Trägermaterial nach einem der Ansprüche 4 bis 10.

12. Wundbehandlungsprodukt nach Anspruch 11, das weiterhin eine Komponente oder eine Zusammensetzung zur Behandlung einer Wunde und/oder ein Therapeutikum umfasst.

13. Medizinisches Diagnostikum, umfassend ein teilchenförmiges Trägermaterial nach einem der Ansprüche 4 bis 10 und umfassend ein Funktionsmaterial, gebunden oder fixiert durch das Trägermaterial.

14. Medizinisches Diagnostikum nach Anspruch 13, wobei das Funktionsmaterial ein von dem Polymer getragenes Enzym umfasst.

15. Verwendung eines teilchenförmigen Trägermaterials nach einem der Ansprüche 4 bis 10 in einem Verfahren, ausgewählt aus Festphasensynthese von Peptiden, Oligonucleotiden, Oligosacchariden; Festphasenextraktion; organischer Festphasenchemie; Immobilisierung einer Spezies, ausgewählt aus Festphasenreagenzien, Metall- und anderen Katalysatoren, Biokatalysatoren, Enzymen, Proteinen, Antikörpern, umfassend polyklonale und monoklonale Antikörper, ganzen Zellen und Polymeren; Zellkultivierung; Bereitstellung einer stationären Phase für die chromatographische Trennung; oder für die Verwendung als Absorptionsmittel oder als biologisch abbaubarer Träger für ein Produkt mit kontrollierter Freisetzung.

## Revendications

1. Polymère de poly-ε-lysine réticulée comprenant de la poly-ε-lysine et un agent de réticulation liés par des liaisons amides, dans lequel l'agent de réticulation comprend deux groupes acide carboxylique ou plus et une chaîne de liaison aliphatique liant les deux groupes ou plus adaptés à réagir avec une amine sur le carbone alpha de la poly-ε-lysine.

2. Polymère selon la revendication 1, qui est insoluble dans l'eau et autres solvants.

3. Polymère selon l'une quelconque de la revendication 1 ou de la revendication 2, sous forme particulaire.

4. Support particulaire comprenant un polymère de poly-ε-lysine réticulée selon l'une quelconque des revendications précédentes.

5. Support particulaire selon la revendication 4, dans lequel le support de poly-ε-lysine réticulée est une particule sphérique, ellipsoïde ou irrégulière.

6. Support particulaire selon la revendication 4 ou la revendication 5, dans lequel le support de poly-ε-lysine réticulée est utilisé pour revêtir une particule directement ou indirectement.

7. Support particulaire selon l'une quelconque des revendications 4 à 6, dans lequel le support de poly-ε-lysine réticulée est utilisé pour revêtir et est lié de façon covalente à une particule.

8. Support particulaire selon l'une quelconque des revendications 4 à 7, dans lequel la poly-ε-lysine réticulée est utilisée pour revêtir une particule polymère organique.

9. Support particulaire selon l'une quelconque des revendications 4 à 7, dans lequel la poly-ε-lysine réticulée est utilisée pour revêtir une particule polymère inorganique.

10. Support particulaire à base de poly-ε-lysine réticulée selon l'une quelconque des revendications 4 à 9, dans lequel la poly-ε-lysine réticulée est fonctionnalisée pour fournir un matériau comprenant un catalyseur, une espèce initiatrice pour la synthèse de peptides, une espèce initiatrice pour la synthèse d'oligonucléotides, une espèce initiatrice pour la synthèse organique en phase solide, un principe actif pharmaceutique, un principe actif agrochimique, une surface pour la séparation chromatographique, une espèce pour promouvoir la culture ou la différentiation cellulaire, une protéine ou autre macromolécule biologique.

11. Produit pour le traitement des plaies comprenant un polymère selon l'une quelconque des revendications 1 à 3 ou un support particulaire selon l'une quelconque des revendications 4 à 10.

12. Produit pour le traitement des plaies selon la revendication 11, qui comprend en outre un composant ou une composition pour le traitement d'une plaie et/ou un agent thérapeutique.

13. Diagnostic médical comprenant un support particulaire selon l'une quelconque des revendications 4 à 10 et comprenant un matériau fonctionnel lié ou retenu par le support.

14. Diagnostic médical selon la revendication 13, dans lequel le matériau fonctionnel comprend une enzyme supportée par le polymère.

15. Utilisation d'un support particulaire selon l'une quelconque des revendications 4 à 10 dans un procédé sélectionné parmi la synthèse en phase solide de peptides, d'oligonucléotides, d'oligosaccharides ; l'extraction en phase solide ; la chimie organique en phase solide ; l'immobilisation d'une espèce sélectionnée parmi des réactifs en phase solide, des catalyseurs de métal et autres, des biocatalyseurs, des enzymes, des protéines, des anticorps comprenant des anticorps polyclonaux et monoclonaux, des cellules entières et des polymères ; la culture cellulaire ; la préparation d'une phase stationnaire pour la séparation chromatographique ; ou pour l'utilisation en tant qu'absorbant ou en tant que vecteur biodégradable pour un produit à libération contrôlée.
